# EUROPEAN PATENT APPLICATION

(11) **EP 1 693 447 A1**
(43) Date of publication of application: **23.08.2006**
(21) Application number: 05425083.2
(22) Date of filing: 18.02.2005
(51) Int. Cl.: C12N 9/02, C12N 1/16, C12N 1/18

(54) **Procedure for the preparation of superoxide dismutase**

(71) Applicant: Gnosis S.p.A., 20033 Desio (MI) (IT)
(72) Inventor: Benedetti, Alberto, 20063 Cernusco Sul Naviglio (MI) (IT); Daly, Simona, 20052 Monza (MI) (IT); Xaiz, Roberto, 20052 Monza (MI) (IT)
(74) Representative: Minoja, Fabrizio

(57) **Abstract**

Disclosed is a process for the preparation of superoxide dismutase which comprises the aerobic culture of a strain of yeast selected from

| |
|---|
| *Kluyveromyces marxianus* L3 GN2255-**NCYC** 3260 |
| *Saccharomyces cerevisiae* L10 GN2256-**NCYC** 3263 |
| *Saccharomyces cerevisiae* L32 GN2257-**NCYC** 3262 |
| *Pichia guillermondi* L13 GN2258-**NCYC** 3261 |

deposited on 19.11.2004 at the National Collection of Yeast Cultures (NCYC), and the extraction of superoxide dismutase from the cells.

## Description

The present invention relates to the production of superoxide dismutase (SOD) by fermentation, using yeasts.

The invention also relates to new strains of yeast, deposited in accordance with the Treaty of Budapest, which have a high SOD production capacity under suitable culture conditions.

### Prior art

All the cells that make up the human body are subject to the toxicity of radical species such as the superoxide radical (Or₂⁻), which are defined as Reactive Oxygen Species (ROS); they are generated by normal metabolic activity and in situations of stress as a result of the formation of products of reduction or excited singlet states. Highly oxidising radical species like ROS damage the cell structures according to different mechanisms: by altering the structure, fluidity and permeability of the membrane and thus interfering with inter-cell communication or damaging the genetic material, which leads to cell death. As the chemical reactivity of the free radicals consequently damages all types of cell macromolecules, if ROS generation is not balanced by adequate detoxification, the result is a situation of oxidative stress, correlated with the etiology of degenerative diseases associated with aging processes.

The superoxide dismutases constitute a single family of enzymes whose only function seems to be rapid elimination of the superoxide anion (O₂⁻), which is biologically generated through a dismutation reaction that produces O₂ and H₂O₂ and is so toxic to the body that its rapid, efficient removal is of vital importance.

All the SODs are metal enzymes whose active site contains a redox-active transition metal ion (copper, iron, manganese or zinc). Cu, Zn-SOD is mainly located in the eukaryotic cells at cytosol level; Mn-SOD is widely distributed between the prokaryotic and eukaryotic organisms, and has also been isolated from yeasts. Fe-SOD is found at cytosol or organelle level in the prokaryotes and in some eukaryotes; and a fourth SOD (Ni-SOD) has also been characterised in some streptomycetes (Kim *et al.,* 1998). Cu, Zn-SODs are homodimers with a molecular weight of approx. 32,000 daltons. The two identical sub-units are associated solely with non-covalent interactions, and each sub-unit binds one copper and one zinc atom. SODs containing manganese and iron have a molecular weight of approx. 23,000 daltons; Fe-SOD seems to exist in dimeric form, whereas Mn-SOD forms both dimers and tetramers (Parker *et al.,* 1984).

There are various types of SOD currently on the market (Table 1):

**Table 1**

| |
|---|
| Bovine liver superoxide dismutase - ammonium sulphate suspension - 3,000 units/mg of protein |
| Bovine erythrocyte superoxide dismutase - lyophilised powder - 2,500-7,000 units/mg of protein |
| Bovine liver superoxide dismutase - freeze-dried powder - 2,500-3,000 units/mg of protein |
| Bovine liver superoxide dismutase - freeze-dried powder - 2,000-6,000 units/mg of protein |
| Canine erythrocyte superoxide dismutase - freeze-dried powder - 2,000-6,000 units/mg of protein |
| *Escherichia coli* superoxide dismutase - freeze-dried powder ≥ 2.500 units/mg of protein |
| *Escherichia coli* superoxide dismutase - freeze-dried powder = 2,500-5,000 units/mg of protein |
| Horseradish superoxide dismutase - freeze-dried powder - 1,000-4,000 units/mg of protein |
| Human erythrocyte superoxide dismutase - freeze-dried powder = 99% =1,350 units/mg of protein |
| Bovine liver - PEG superoxide dismutase - freeze-dried powder ≥ 1,350 units/mg of protein |
| Superoxide dismutase - CME *Cucumis melo* LC. Extract - stabilised with gliadin biopolymers 95± 8 units/mg (1) |

| |
|---|
| (1) patent: US Patent 5,747,043 |

SOD is widely found in nature, and is produced by the majority of cells that metabolise oxygen, namely plants, animals and micro-organisms. Among the micro-organisms it is very common in fungi and aerobic, microaerophilic and anaerobic bacteria. Aerobic micro-organisms possess superoxide dismutase to prevent biological damage due to the cytotoxic, mutagenic reactive oxygen species (ROS) that form during normal aerobic catabolism. In microaerophilic micro-organisms such as lactic bacteria, which present a certain tolerance to oxygen, significant quantities of superoxide radicals can accumulate during development in the presence of oxygen. The NADH oxidase/peroxidase system controls the intracellular redox potential and catalyses the conversion of hydrogen peroxide to water. The ability to withstand oxidative stress varies according to the groups of lactic bacteria, and is determined by various biochemical mechanisms including the presence of superoxide dismutase. SOD appears to be present in many species of *Lactobacillus,* but in low quantities (9-15 U/mg of protein). However, the productivity of SOD in various species of yeast is higher, especially in *Saccharomyces* and *Kluyveromyces,* which are food grade yeast species classified by the Food and Drug Administration as "Generally Regarded As Safe" (GRAS).

A number of studies have been and are being conducted to define the role of SODs as enzymes of great medical importance, as they are potential therapeutic agents in disorders correlated with oxidative stress.

Examples of the role of SOD in some oxygen-mediated biological reactions that underlie its therapeutic potential at molecular and cell level in relation to human disorders are reported below (Table 2).

**Table 2**

| **SYSTEM** | **RESPONSE** | |
|---|---|---|
| 1. Molecular | SOD inhibits auto-oxidation of epinephrine, vitamin C₂ and the redox metabolism of adriamycin, daunorubicin and mitomycin C. | |
| 2. Cellular | (i) | SOD inhibits the introduction of chromosome aberrations into human lymphocytes by phorbol myristate acetate |
| | (ii) | SOD inhibits mutagenesis induced by phagocytosis |
| | (iii) | SOD radioprotects the progenitor cells of the bone marrow |
| | (iv) | SOD, injected intravenously, invalidates myelosuppression induced by chemotherapy |
| | (v) | In leukaemia, the SOD content of the erythrocytes is often low, and high levels of methaemoglobin and malondialdehyde contribute to reducing life expectancy and the onset of anaemia |
| | (vi) | SOD and catalase inhibit the cytotoxicity of the low-density lipoproteins against fibroblasts and endothelial cells. The protection of SOD is proportional to its intracellular concentration, which can be increased with liposomes |
| | (vii) | SOD and catalase inhibit phagocytic bactericidal activity |
| 3. Body | (i) | *P. berghei* causes the release of O₂ from the phagocytic cells and adopts the SOD of the host cell. |
| | (ii) | SOD inhibits *Trichinella spiralis* infection caused by O₂ |

### Description of the invention

Strains of *Pichia, Saccharomyces* and *Kluyveromyces,* which present high levels of SOD activity and can therefore be advantageously used to produce SOD by fermentation, have now been found, following a screening designed to select food grade yeast species classified by the Food and Drug Administration come "Generally Regarded As Safe" (GRAS).

The strains, which constitute a first subject of the invention, were deposited in the National Collection of Yeast Cultures, Norwich, GB (NCYC), on 19.11. 2004, and are reported in Table 3.

**Table 3**

| |
|---|
| *Kluyveromyces marxianus* L3 GN2255-**NCYC** 3260 |
| *Saccharomyces cerevisiae* L10 GN2256-**NCYC** 3263 |
| *Saccharomyces cerevisiae* L32 GN2257-**NCYC** 3262 |
| *Pichia guillermondi* L13 GN2258-**NCYC** 3261 |

Superoxide dismutase activity proved particularly high in *Kluyveromyces marxianus* L3 GN2255-**NCYC** 3260 and *Saccharomyces cerevisiae* L10 GN2256-**NCYC** 3263.

The biomass is prepared according to well-known, established fermentation techniques: the yeast cultures were developed aerobically in YPG complex medium, minimal synthetic medium or semi-synthetic medium. The culture media consisted of carbon sources (as glucose, fructose, FOS, saccharose, lactose, starch, sugar beet or sugar cane molasses, lactic acid, citric acid, acetic acid, sodium acetate, ethanol, glycerol, etc.); sources of nitrogen (as urea, ammonia, ammonium sulphate, yeast extract or autolysate, peptone, etc.); and various salts (as mono- and dibasic potassium phosphate, NH₄ sulphate, NH₄ phosphate, calcium chloride, Mg sulphate, Mn sulphate, Zn sulphate, NaCl, etc.). The pH is between 3 and 9, and more precisely between 4.5 and 8.0; the air flow is between 0.25 and 2.5 vvm, and more precisely between 0.5 and 1.2 vvm; the agitation speed in the bioreactor is between 300 and 1000 rpm, and more precisely between 350 and 850 rpm; the pressure in the bioreactor is between 0.1 and 3 bars, more precisely between 0.3 and 2.0 bars, and preferably from 1 to 2.0 bars; the size of the Erlenmeyer flasks used for the cultures is between 100 ml and 2.5 1. Fermentation is conducted in batch, repeated-batch, continuous or fed-batch mode; traditional fermenters (STR or CSTR) or airlift fermenters are used. In continuous mode the dilution rate is between 0.02 and 0.5 h⁻¹, and more precisely between 0.03 and 0.3 h⁻¹. In fed-batch mode the dilution rate is between 0.01 and 1.0 h⁻¹. According to a preferred aspect of the invention, operations are performed in the presence of hydrogen peroxide at concentrations in the culture medium of between 10 and 60 mM, and more precisely between 20 and 55 mM. A particularly preferred carbon source consists of glycerol, glucose ethanol or lactate, and more preferably glycerol.

The biomass is collected by centrifugation or microfiltration, washed once or twice with 0.05 M K-phosphate EDTA buffer at pH 7.8 and resuspended in that buffer. A protease inhibitor (Amersham Biosciences) is added to the suspension; cell rupture is effected by sonication, heat or acid treatment, enzymatic lysis, or mechanical breakage with glass balls, a mortar, abrasive mill, homogeniser or French press. The test for the quantitation of proteins and evaluation of specific SOD activity is then performed on the cell extract collected by centrifugation. The protein concentration of the extracts is determined with the Lowry test (Lowry *et al.,* 1951), the Bradford test (Bradford Protein Assay, BIORAD) or a commercial kit (Protein Assay kit, BIORAD, Calbiochem, Cayman Chemical, Novage, etc.). The extract is dialysed at +4°C overnight against 0.05 M K-phosphate EDTA buffer at pH 7.8, using the mini-dialysis kit with cut-off of 1 or 8 Kda (Amersham Biosciences). SOD enzymatic activity is measured by three different methods:
1. the "SOD Assay Kit - WST/Dojindo Molecular Technologies, Inc.", based on the Beauchamp and Fridovich protocol (1971)
2. the assay that uses enzymatic inhibition of epinephrine oxidation (Hara *et al.,* 1971);
3. the assay that uses enzymatic inhibition of pyrogallol oxidation (Hara *et al.,* 1974).

The dry weight of the biomass is determined by filtration of the cells contained in aliquots of fermentation broth on cellulose nitrate membrane filters (0.45 µm) previously dried and weighed.

The invention is illustrated in greater detail in the examples below.

### EXAMPLE 1

*Kluyveromyces marxianus* L3 GN2255-**NCYC** 3260 is cultivated aerobically in 2 1 baffled Erlenmeyer flasks agitated at 155 rpm on a rotary shaker thermostated at +30°C, in YPG complex medium having the following composition (g L⁻¹):

| | |
|---|---|
| Glucose | 20 |
| Casein pancreatic hydrolysed peptone | 20 |
| Yeast extract | 10 |

The pH of the medium is acidified with 0.5 M HCl to pH 7.

The medium is sterilised at 110°C for 30 minutes: the glucose is autoclaved (110°C for 30 minutes) separately and added to the sterile basic medium.

The biomass is collected by centrifugation or microfiltration and then washed once or twice with 0.05 M K-phosphate EDTA buffer at pH 7.8 and resuspended in that buffer. Cell rupture is effected by mechanical breakage with glass beads. The test for protein quantitation and evaluation of specific SOD activity is then performed on the cell extract collected by centrifugation.

The productivity kinetics of the SOD enzyme present a peak of productivity at the end of the lag-phase, followed by a decline during the exponential growth stage; peak productivity, corresponding to a specific activity of 55 U/(mg protein), is observed approx. one hour after the steady state is reached.

### EXAMPLE 2

As in example 1, where the micro-organism *Kluyveromyces marxianus* L3 GN2255-**NCYC** 3260 is replaced with *Saccharomyces cerevisiae* L10 GN2256-**NCYC** 3263. SOD activity is analysed on the cell extract prepared as described in example 1. The maximum specific activity of SOD is 47 U/(mg of protein), and is observed approx. one hour after the steady state is reached.

### EXAMPLE 3

As in example 1, where the micro-organism *Kluyveromyces marxianus* L3 GN2255-**NCYC** 3260 is replaced with the yeast *Pichia guillermondi* L13 GN2258-**NCYC** 3261. SOD activity is analysed on the cell extract prepared as described in example 1. The maximum specific activity of SOD is 25 U/(mg of protein), and is observed approx. one hour after the steady state is reached.

### EXAMPLE 4

As in example 1, where the micro-organism *Kluyveromyces marxianus* L3 GN2255-**NCYC** 3260 is replaced with the yeast *Saccharomyces cerevisiae* L32 GN2257-**NCYC** 3262. SOD activity is analysed on the cell extract prepared as described in example 1. The maximum specific activity of SOD is 30 U/(mg of protein), and is observed approx. one hour after the steady state is reached.

### EXAMPLE 5

*Kluyveromyces marxianus* L3 GN2255-**NCYC** 3260 is cultivated aerobically in YPG complex medium in batch mode in a 2 L bioreactor, under the following operating conditions: agitation speed 400 rpm, air flow 1 vvm, overpressure 0.3 bars. SOD activity is analysed on the cell extract prepared as described in example 1. A SOD activity peak of approx. 50 U/ (mg protein) was observed approx. 2 hours after the steady state was reached.

### EXAMPLE 6

As in example 1, where the micro-organism *Kluyveromyces marxianus* L3 GN2255-**NCYC** 3260 is replaced with *Saccharomyces cerevisiae* L10 GN2256-**NCYC** 3263. The culture of *Saccharomyces cerevisiae* L10 GN2256-**NCYC** 3263 is grown as described in example 5. SOD activity is analysed on the cell extract prepared as described in example 1. A SOD activity peak of approx. 40 U/ (mg protein) was observed approx. 2 hours after the steady state was reached.

### EXAMPLE 7

*Kluyveromyces marxianus* L3 GN2255-**NCYC** 3260 is cultivated aerobically in YPG complex medium in continuous mode in a 2 1 **CSTR** bioreactor, under the following operating conditions: agitation speed 400 rpm, air flow 1 vvm, overpressure 0.3 bars, dilution rate 0.2 h⁻¹. SOD activity is analysed on the cell extract prepared as described in example 1. In the steady state, the specific activity of SOD is 45 U/mg protein.

### EXAMPLE 8

As in example 7, but varying the overpressure to 1.2 bars. In the steady state, the specific activity of SOD is 70 U/mg protein.

### EXAMPLE 9

As in example 7, but varying the dilution rate to 0.07 h⁻¹. In the steady state, the specific activity of SOD is 145 U/mg protein.

### EXAMPLE 10

As in examples 8 and 9, but adding 50 mM hydrogen peroxide H₂O₂ to the culture broth. In the steady state, the specific activity of SOD is 255 U/mg protein.

### EXAMPLE 11

As in example 7, using a minimal medium with the following composition (g 1⁻¹):

| | |
|---|---|
| Glucose | 20 |
| KH₂PO₄ | 9.52 |
| K₂HPO₄ | 3.48 |
| CaCl₂ | 0.001 |
| (NH₄)₂SO₄ | 5 |
| MgSO₄ | 0.5 |
| NaCl | 0.1 |
| Boric acid | 0.5 |
| CuSO₄ | 0.04 |
| Sodium molybdate | 0.2 |
| ZnSO₄ | 0.4 |
| KI | 0.1 |
| FeCl₃ | 0.2 |
| MnSO₄ | 0.4 |
| Biotin | 0,002 |
| Calcium pantothenate | 0.4 |
| Folic acid | 0,002 |
| Niacin | 0.4 |
| PABA | 0.2 |
| Pyridoxine | 0.4 |
| Riboflavin | 0.2 |
| Thiamine | 0.4 |

The pH of the medium is buffered to 6.3 due to the presence of mono- and dibasic phosphate at the total concentration of 0.1 M.

The medium is sterilised at 110°C for 30 minutes; the glucose is autoclaved (110°C for 30 minutes) separately and added to the sterile basic medium.

In the stationary phase, the specific activity of SOD is 50 U/mg protein.

### EXAMPLE 12

As in example 8, using the minimal medium described in example 11. In the steady state, the specific activity of SOD is 90 U/mg protein.

### EXAMPLE 13

As in example 9, using the minimal medium described in example 11. In the steady state, the specific activity of SOD is 250 U/mg protein.

### EXAMPLE 14

As in example 10, using the minimal medium described in example 11. In the steady state, the specific activity of SOD is 500 U/mg protein.

### EXAMPLE 15

*Kluyveromyces marxianus* L3 GN2255-NCYC 3260 is cultivated aerobically in batch mode in a 2 L STR bioreactor under the following operating conditions: agitation speed 400 rpm, air flow 1 vvm, overpressure 0.3 bars. The SOD activity test is performed on the cell extract prepared as described in example 1. The synthetic medium used has the following composition (g l⁻¹):

| | |
|---|---|
| Glycerol | 20 |
| KH₂PO₄ | 9.52 |
| K₂HPO₄ | 3.48 |
| CaCl₂ | 0.001 |
| (NH₄)₂SO₄ | 5 |
| MgSO₄ | 0.5 |
| NaCl | 0.1 |
| Boric acid | 0.5 |
| CuSO₄ | 0.04 |
| Sodium molybdate | 0.2 |
| ZnSO₄ | 0.4 |
| KI | 0.1 |
| FeCl₃ | 0.2 |
| MnSO₄ | 0.4 |
| Yeast autolysate | 5 |

The pH of the medium is buffered to 6.3 due to the presence of mono- and dibasic phosphate at the total concentration of 0.1 M.

The medium is sterilised at 110°C for 30 minutes; the glycerol is autoclaved (110°C for 30 minutes) separately and added to the sterile basic medium.

In the stationary phase, the specific activity of SOD is 1200 U/mg protein.

### EXAMPLE 16

As in example 15, but varying the overpressure to 1.2 bars. In the stationary phase, the specific activity of SOD is 1400 U/mg protein.

### EXAMPLE 17

As in example 15, but adding 50 mM hydrogen peroxide H₂O₂ to the culture broth. In the stationary phase, the specific activity of SOD is 2000 U/mg protein.

### EXAMPLE 18

*Kluyveromyces marxianus* L3 GN2255-**NCYC** 3260 is cultivated aerobically in semi-synthetic medium as described in example 15, in continuous mode in a 2 L CSTR bioreactor, under the following operating conditions: agitation speed 400 rpm, air flow 1 vvm, overpressure 1.2 bars, dilution rate 0.07 h⁻¹. The SOD activity test is performed on the cell extract prepared as described in example 1. In the steady state, the specific activity of SOD is 2500 U/mg protein.

### EXAMPLE 19

*Kluyveromyces marxianus* L3 GN2255-**NCYC** 3260 is cultivated aerobically in semi-synthetic medium as described in example 15, in fed-batch mode in a 2 L STR bioreactor, under the following operating conditions: agitation speed 400 rpm, air flow 1 vvm, overpressure 0.3 bars. The carbon source used at the batch stage preceding the start of the feed is glucose (20 gl-¹). The carbon source used at the feed stage is glycerol (20 gl⁻¹). The volumetric flow rate of the feed increases exponentially from 0.07 h⁻¹.

The SOD activity test is performed on the cell extract prepared as described in example 1.

In the steady state, the specific activity of SOD is 3000 U/mg protein.

### EXAMPLE 20

*Kluyveromyces marxiarius* L3 GN2255-**NCYC** 3260 is cultivated aerobically in batch mode in semi-synthetic medium as described in example 15, in a 20 L STR bioreactor, under the following operating conditions: agitation speed 230 rpm, air flow 1 vvm, overpressure 0.4 bars. The SOD activity test is performed on the cell extract prepared as described in example 1.

In the stationary phase, the specific activity of SOD is 1250 U/mg protein.

### EXAMPLE 21

*Kluyveromyces marxianus* L3 GN2255-**NCYC** 3260 is cultivated aerobically in batch mode in semi-synthetic medium as described in example 15, in a 300 L STR bioreactor, under the following operating conditions: agitation speed 750 rpm, air flow 2 vvm, overpressure 0.4 bars. The SOD activity test is performed on the cell extract prepared as described in example 1.

In the stationary phase, the specific activity of SOD is 850 U/mg protein.

### EXAMPLE 22

As in example 21, but adding 50 mM hydrogen peroxide H₂O₂ to the culture broth. In the stationary phase, the specific activity of SOD is 1150 U/mg protein.

### EXAMPLE 23

As in example 21, but varying the overpressure to 1.2 bars. In the stationary phase, the specific activity of SOD is 1000 U/mg protein.

### EXAMPLE 24

*Kluyveromyces marxianus* L3 GN2255-**NCYC** 3260 is cultivated aerobically in semi-synthetic medium as described in example 15, in batch mode in a 50 L airlift bioreactor, under the following operating conditions: pneumatic agitation with air flow in a range between 0.0126 and 0.0440 ms⁻¹, overpressure 0.4 bars, temperature 30°C. The SOD activity test is performed on the cell extract prepared as described in example 1.

In the stationary phase, the specific activity of SOD is 125 U/mg protein.

### EXAMPLE 25

As in example 15, where the micro-organism *Kluyveromyces marxianus* L3 GN2255-**NCYC** 3260 is replaced by *Saccharomyces cerevisiae* L10.

In the stationary phase, the specific activity of SOD is 300 U/mg protein.

### EXAMPLE 26

Non-proliferating cells of *Kluyveromyces marxianus* L3 GN2255-**NCYC** 3260 are incubated in the presence of 50 mM H₂O₂ , O₂ flow of 1 vvm, and 1.2 bars pressure in the head space.

The specific activity of SOD is 250 U/mg of protein.

### EXAMPLE 27

*Kluyveromyces marxianus* L3 GN2255-**NCYC** 3260 is cultivated aerobically in semi-synthetic medium as described in example 15, in batch mode in a 2 L STR bioreactor, under the following operating conditions: agitation speed 400 rpm, air flow 1 vvm, overpressure 1.2 bars. The carbon source used at the feed stage is ethanol (20 gl⁻¹). The SOD activity test is performed on the cell extract prepared as described in example 1. In the steady state, the specific activity of SOD is 2500 U/mg protein.

### EXAMPLE 28

*Kluyveromyces marxianus* L3 GN2255-**NCYC** 3260 is cultivated aerobically in semi-synthetic medium as described in example 15, in batch mode in a 2 L STR bioreactor, under the following operating conditions: agitation speed 400 rpm, air flow 1 vvm, overpressure 1.2 bars. The carbon source used at the feed stage is lactate (20 gl⁻¹). The SOD activity test is performed on the cell extract prepared as described in example 1. In the steady state, the specific activity of SOD is 2500 U/mg protein.

## Claims

1. Process for the preparation of superoxide dismutase which comprises the aerobic culture of a strain of yeast selected from
| |
|---|
| *Kluyveromyces marxianus* L3 GN2255-**NCYC** 3260 |
| *Saccharomyces cerevisiae* L 10 GN2256-**NCYC** 3263 |
| *Saccharomyces cerevisiae* L32 GN2257-**NCYC** 3262 |
| *Pichia guillermondi* L13 GN2258-**NCYC** 3261 |
deposited on 19.11.2004 at the National Collection of Yeast Cultures (NCYC), and the extraction of superoxide dismutase from the cells.

2. Process as claimed in claim 1, wherein the strain is *Kluyveromyces marxianus* no. GN2255, deposited in the NCYC collection under access no. 3260.

3. Process as claimed in claim 1, wherein the strain is *Saccharomyces cerevisiae* no. GN2256, deposited in the NCYC collection under access no. 3263.

4. Process as claimed in claim 1, wherein the strain is *Pichia guillermondi* no. GN2258, deposited in the NCYC collection under access no. 3261.

5. Process as claimed in claim 1, wherein the strain is *Saccharomyces cerevisiae* no. GN2257, deposited in the NCYC collection under access no. 3262.

6. Process as claimed in any of claims 1 to 5, wherein ethanol, glucose, lactate or glycerol is used as carbon source.

7. Process as claimed in claim 6, wherein glycerol is used as carbon source.

8. Process as claimed in any of claims 1 to 7, wherein the culture medium contains H₂O₂ at the concentration of between 1 mM and 300 mM.

9. Process as claimed in any of claims 1 to 8, wherein operations are performed at a head pressure of between 0.4 and 1.5 bars.

10. Process as claimed in any of claims 1 to 9, **characterised in that** operations are performed in continuous mode with a dilution rate of between 0.02 and 0.4 h⁻¹.

11. Process as claimed in any of claims 1 to 10, wherein fermentation is performed in an STR, CSTR or airlift fermenter.

12. Process as claimed in any of claims 1 to 10, wherein fermentation is performed in batch or fed-batch mode.

13. Process as claimed in any of claims 1 to 12, **characterised in that** non-proliferating cells are used.

14. Strains of yeast selected from
| |
|---|
| *Kluyveromyces marxianus* L3 GN2255-**NCYC** 3260 |
| *Saccharomyces cerevisiae* L10 GN2256-**NCYC** 3263 |
| *Saccharomyces cerevisiae* L32 GN2257-**NCYC** 3262 |
| *Pichia guillermondi* L13 GN2258-**NCYC** 3261 |
deposited on 19.11.2004 at the National Collection of Yeast Cultures (NCYC).
